# EUROPÄISCHE PATENTSCHRIFT

(11) **EP 2 218 456 B1**
(45) Veröffentlichungstag und Bekanntmachung des Hinweises auf die Patenterteilung: **16.03.2011**
(21) Anmeldenummer: 09162527.7
(22) Anmeldetag: 11.06.2009
(51) Int. Cl.: A61K 38/16

(54) **Lektinzusammensetzung zur Prophylaxe und/oder Behandlung von unerwünschten Arzneimittelnebenwirkungen**
Lectin composition for the prophylaxis and/or treatment of undesired drug side effects
Composition de lectine pour la prophylaxie et/ou le traitement d'effets secondaires indésirables d'un médicament

(30) Priorität: 10.02.2009 DE 102009008313
(43) Veröffentlichungstag der Anmeldung: 18.08.2010
(73) Patentinhaber: Helvista AG, 6300 Zug (CH)
(72) Erfinder:
(74) Vertreter: Wagner, Sigrid

(56) Entgegenhaltungen:
- EP-A1- 2 106 705
- WO-A1-97/49420
- WO-A1-99/11278
- Kyberg Pharma Vertriebs-GmbH & Co. KG: "Equizym MCA" XP002583694 Gefunden im Internet: URL:http://www.apomio.de/packungsbeilage/6 640019_p.pdf> [gefunden am 2010-05-25]
- Beuth J: "Komplementärmedizin" 25. April 2007 (2007-04-25), XP002583695 Gefunden im Internet: URL:http://www.tumorzentrum-bonn.de/pdf/Pa tiententag3/Beuth.pdf> [gefunden am 2010-05-25]
- "Index of /pdf/Patiententag3" XP002584303 Gefunden im Internet: URL:http://www.tumorzentrum-bonn.de/pdf/Pa tiententag3> [gefunden am 2010-05-27]

## Beschreibung

Die Erfindung betrifft eine Lektinzusammensetzung zur Prophylaxe oder/und Behandlung von Aromatase-Hemmer -, Antiöstrogen - oder/und GnRH-Analoga - bedingten unerwünschten Arzneimittelnebenwirkungen oder von durch einen Hormondefizit - bedingten Beschwerdekomplex in einem Individuum.

Die Antihormontherapie ist eine wichtige adjuvante Therapiemaßnahme bei hormonrezeptorpositiven Krebsarten, z.B. Brustkrebs oder Prostatakrebs. Diese beiden Krebsarten sind auch in Deutschland mit ca. 50 000 - 60 000 bzw. ca. 60 000 Neuerkrankungen pro Jahr führend. Wenn sich auf den Krebszellen Rezeptoren für Hormone wie Östrogen und Progesteron beim Brustkrebs bzw. Testosteron beim Prostatakrebs befinden, regen diese Hormone den Krebs zum Wachstum an. Ein derartiger Wachstumsreiz durch Hormone kann durch eine Blockade oder durch eine Hemmung der Bildung der Hormone verhindert werden. Voraussetzung ist, dass der Krebs hormonrezeptorpositiv ist. Bei 80% aller Brust- und ProstataPatienten ist dies der Fall. Eine derartige antihormonelle Therapie, die laut Behandlungsleitlinien über 5 Jahre und in besonderen Fällen auch länger durchgeführt werden soll, reduziert signifikant das Risiko der Rezidivierung und Metastasierung, wodurch die Überlebensrate der Patientinnen deutlich erhöht wird.

Zur hormonellen, d.h. anti-hormonellen, Behandlung von Brustkrebs stehen beispielsweise drei Therapieoptionen zur Verfügung:
1. Antiöstrogene wie Tamoxifen: sie verdrängen als Gegenspieler das körpereigene Östrogen von den Hormonrezeptoren der Krebszelle. Antiöstrogene senken das Risiko einer Wiedererkrankung bei Frauen aller Altersgruppen signifikant.
2. Aromatasehemmer wie Femara, Aromasin, Arimidex: sie verhindern den Aufbau von komplettem, funktionsfähigem Östrogen durch Hemmung des Enzyms Aromatase, welches den letzten Schritt der Östrogensynthese vollzieht. Somit steht das entsprechende Östrogen für den Körper nicht zur Verfügung und die entsprechenden Hormonrezeptoren werden nicht aktiviert.
3. GnRH (Gonadotropin Releasing Hormone) - Analoga wie Zoldex: derartige Analoga blockieren in der Hypophyse die Bildung des Hormons FSH (Follicle Stimulating Hormone), das die Eierstöcke zur Produktion von Östrogen anregt.

Verbunden mit dieser Behandlung sind allerdings erhebliche und vielschichtige unerwünschte Arzneimittelnebenwirkungen. Unerwünschte Arzneimittelnebenwirkungen werden auch bezeichnet als unerwünschte arzneimittelbedingte Nebenwirkungen und werden im Folgenden gleichbedeutend verwendet. Derartige Nebenwirkungen sind sehr gravierend für die Patientinnen und stellen eine drastische Einschränkung der Lebensqualität (quality of life, QoL) dar. Es kommt zu einem Austrocknen der Haut und Schleimhäute mit den damit verbundenen unangenehmen Begleiterscheinungen für die Betroffenen wie bspw. Hautjucken, trockener Mund, Augenbrennen/-schmerzen und der Unmöglichkeit weiterhin Kontaktlinsen zu tragen. Betroffen ist allerdings der gesamte Schleimhautkomplex umfassend Augen-, Nasen-, Mund-, Rachen-, Vaginalschleimhäute sowie der Magen-Darm-Trakt. Weitere negative Begleiterscheinungen einer derartigen Behandlung sind Gelenkschmerzen, die nicht nur überaus schmerzhaft sind, sondern vor allem das tägliche Leben der Patientinnen erheblich einschränken. Insbesondere der morgendliche "Anlaufschmerz" wirkt sich überaus unangenehm aus. Er betrifft z.B. das Unvermögen aufgrund der Gelenkschmerzen die morgendliche und tägliche Arbeit der Kinderbetreuung zu leisten. Aufgrund der Komplexität der unerwünschten Arzneimittelnebenwirkungen ist vielfach das gesamte familiäre Umfeld betroffen mit den damit verbundenen gravierenden Belastungen für das tägliche Leben. Darüber hinaus haben die unerwünschten Arzneimittelnebenwirkungen psycho-soziale Implikationen, die weit in die Privatsphäre reichen, z.B. Probleme im Sexualbereich verursacht durch Vaginaltrockenheit**.** Die unerwünschten Arzneimittelnebenwirkungen führen zu einer so starken Belastung für das gesamte Leben und die Lebensqualität der betroffenen Patientinnen, dass die Patientinnen vielfach erwägen, die Antihormontherapie trotz der möglichen negativen medizinischen Konsequenzen abzubrechen. Die unerwünschten Arzneimittelnebenwirkungen stellen in ihrer Gesamtheit somit mehr als nur punktuelle körperliche Probleme dar, sondern sie bedeuten für die Lebensqualität der Betroffenen einen Nebenwirkungskomplex, der über die Einzelsymptome hinausgeht. Dieser wirkt sich nicht nur körperlich aus, sondern wirft vor allem psycho-sozial einschneidende Probleme und Konsequenzen auf. Somit besteht ein Bedürfnis, Mittel zu entwickeln, die derartige unerwünschte Arzneimittelnebenwirkungen helfen zu mildern oder zu beseitigen.

Ähnliche Auswirkungen hat auch ein endogener Östrogendefizit in der Menopause bei Frauen. Das erste Stadium, die sogenannte Perimenopause, ist gekennzeichnet u.a. durch Hitzewallungen, Brustschmerzen, Gewichtszunahme, übermäßige Nervosität sowie Empfindlichkeit. Die Menopause ist zudem gekennzeichnet durch Müdigkeit, Insomnia, Trockenheit der Vaginalschleimhaut sowie Gelenk - und Knochenprobleme und einer erhöhten Rate von Infektionen im urogenitalen Bereich. Weiterhin treten psychische Probleme wie Angstzustände, Schlafstörungen und Schwermut bis zur Depressionen auf. Die Menopause ist aufgrund der vielfältigen Probleme mit starken Belastungen für den Partner bzw. das gesamte familiäre Umfeld über einen Zeitraum von mehreren Jahren verbunden. Die bekannten Behandlungsansätze sind bisher leider unzureichend. Somit besteht auch für diesen Problemkomplex ein starkes Bedürfnis zumindest einen Teil des Symptomkomplexes mit geeigneten Mitteln zu mildern.

Lektine sind zuckerbindende Eiweisse mit hoher Spezifität für definierte Zucker und sie werden entsprechend z. B. in der Analytik und Forschung für die Bestimmung der Zuckerreste von Glykoproteinen eingesetzt. Lektine sind z.B. aus der Mistel bekannt und werden als Mistelextrakttherapie als i.v., s.c. oder i.c. Injektionen KrebsPatientinnen verabreicht. Mistellektine sind beschrieben als zytotoxisch und immunmodulierend.

EP 0 942 741 B1 beschreibt Lektine und ihre Verwendung zur Kontrolle der Schleimhautzellproliferation. Die erfindungsgemäße Verwendung wird in diesem Dokument weder beschrieben noch nahegelegt.

US 2007/0031398 A1 beschreibt Enzym-enthaltende Zusammensetzungen. Die erfindungsgemäßen Zusammensetzungen sind nicht beschrieben. Gleichermassen ist die erfindungsgemäße Verwendung weder beschrieben noch nahegelegt.

Die Erfindung stellt in einem Aspekt eine Lektinzusammensetzung zur Prophylaxe oder/und Behandlung von Aromatase-Hemmer -, Antiöstrogen - oder/und GnRH-Analoga - bedingten unerwünschten Arzneimittelnebenwirkungen oder von durch einen endogenen Östrogendefizit in der Menopause bei Frauen - bedingten Besrhwerdekomplex in einem Individuum bereit.

Die Erfinder haben nun überraschender Weise gefunden, dass Lektinzusammensetzungen vorteilhafter Weise eingesetzt werden können, um die oben genannten negativen Arzneimittelnebenwirkungen zu behandeln. Dabei konnte eine deutliche Verringerung von Symptomen wie Austrocknen der Haut und der Augen-, Nasen-, Mund-, Rachen- und Vaginalschleimhäute mit den damit verbundenen unangenehmen Begleiterscheinungen für die Betroffenen wie bspw. Hautjucken, trockener Mund, Augenbrennen/-schmerzen und der Unmöglichkeit weiterhin Kontaktlinsen zu tragen oder/und Gelenkschmerzen erreicht werden. Gleichermaßen kann die Lektinzusammensetzung bei einer prophylaktischen Behandlung verwendet werden.

Die erfindungsgemäße Lektinzusammensetzung wird vorzugsweise als eine pharmazeutische Zusammensetzung, ein diätetisches Lebensmittel, ein Lebensmittel für die bilanzierte Diät oder/und eine ergänzend bilanzierte Diät (ebD) bereitgestellt. Weiterhin kann die erfindungsgemäße Lektinzusammensetzung ein Medizinprodukt sein.

Bei der jeweiligen Formulierung und Herstellung können die dem Fachmann geläufigen weiteren Hilfs- und Zusatzstoffe sowie Verfahren zum Einsatz kommen.

In einer erfindungsgemäßen Zusammensetzung können sowohl pflanzliche wie tierische Lektine zur Anwendung kommen. Vorzugsweise kann das Lektin oder die Lektine aus einer Hülsenfrucht oder/und einem Nachtschattengewächs stammen. Es kann beispielsweise aus einer Wurzel isoliert sein. In einer weiteren bevorzugten Ausführungsform werden tierische Lektine verwendet, die beispielsweise aus einer Schnecke stammen. Auch Lektine aus Pilzen können eingesetzt werden. Weitere bevorzugte Lektine haben ihren Ursprung in Algen oder Muscheln.

Andere Lektine können auch in der erfindungsgemäßen Zusammensetzung verwendet werden. Beispielsweise können alle pflanzlichen Lektine Verwendung finden. Weitere bevorzugte Lektine stammen aus der Erdnuss (*Arachis hypogaea*), dem Getreide, der Jackbohne (*Canavalia ensiformis*), der Brennessel (*Urtica major*), Soja, der Zwiebel, Linsen, der Tomate, der Kartoffel oder dem Lotuskeimling. Erfindungsgemäß verwendbare Lektine schliessen alle natürlich vorkommenden sowie in jeglichem Masse isolierte Lektine, chemisch synthetisierte, biotechnologisch hergestellte sowie modifizierte Lektine oder natürlich vorkommende oder synthetische Derivate davon ein. Dem Fachmann ist die Herstellung, Isolierung und Reinigung von Lektinen sowie ihre biotechnologische Bereitstellung bekannt (siehe z.B. Pusztai und Palmer, 1977, J. of the Science of Food and Agriculture 28, 620 - 623; Pusztai et al., 1993, J. of Applied Bacteriology 75, 360 - 368; US 4,889,842).

Lektine sind Proteine und somit nur begrenzt stabil. Sie können instabil werden und denaturieren. Dies hängt von einer Reihe von Faktoren ab, wie Wärmeeinwirkung, Einfluss von Säuren oder Basen etc. Einige Lektine sind stabiler als andere und mehr oder weniger resistent gegenüber derartigen Einwirkungen. Die erfindungsgemäße Anwendung der Lektine erfordert ihre Proteineigenschaften und Aktivität. Somit ist ihre wirksame Aktivität von Bedeutung und für den Fachmann ist es offensichtlich, dass es bei der Formulierung bzw. der Einstellung der Tagesdosis auf diese Aktivität ankommt, um die positiven Wirkungen der erfindungsgemäßen Behandlung zu erzielen. Entsprechend wird der Fachmann die entsprechende Darreichungsform und ihre Zubereitung wählen, um die Stabilität des Lektin's während der Lagerung zu gewähren. Er wird gleichermaßen die Zusammensetzung mit geeigneten Hilfs- und Zusatzstoffen wählen, um das Lektin vor einem schädlichen zu niedrigen pH-Wert zu schützen, der z.B. im Magenmilieu vorliegt. Diese Kenntnisse entsprechen auch dem allgemeinen Fachwissen des einschlägigen Fachmann's auf dem hier anwendbaren technischen Gebiet. Die folgenden Angaben zum Lektingehalt bzw. der zu verabreichenden Tagesdosis beziehen sich auf Lektine mit ihren natürlich vorkommenden Eigenschaften.

Die Aktivität der Lektine kann durch chemische oder biotechnologische Modifikationen wie z.B. Mutation der Sequenz oder Veränderung der Lektinsequenz in ihrer Länge (Trunkierung) erhöht werden.

In einer bevorzugten Ausführungsform enthält die Zusammensetzung ein Lektin aus Lens culinaris, Pisum sativum oder/und Phaseolus vulgaris.

Die beschriebenen negativen Arzneimittelnebenwirkungen werden im Allgemeinen durch jegliche Substanz verursacht, die eine Aromatase-Hemmer/Inhibitorfunktion aufweist, ein Antiöstrogen oder ein GnRH-Analogon ist. Die erfindungsgemäße Zusammensetzung kann hier erfolgreich in einer der oben und im weiteren beschriebenen Formen angewendet werden. Beispiele für Aromatase-Hemmer, auch als Aromataseinhibitoren bezeichnet, sind Aminoglutethimid, Anastrozol (Arimidex), Letrozol (Femara), Vorozol (Rivizor), Exemestan (Aromasin), 1,4,6-androstatrien-3,17-dione ATD), Formestan (Lentaron), Testolactone (Teslac) und Fadrozol (Afeman).

Heute verwendete Antiöstrogene oder auch als selektive Östrogenrezeptormodulatoren (SERM) bezeichnete Stoffe sind z.B. Clomifen, Raloxifen, Tamoxifen, Toremifen, Bazedoxifen, Lasofoxifen und Ormeloxifen.

GnRH-Analoga sind beispielsweise aus Leuprorelin (Eligard^{®}, Enantone^{®}), Goserelin (Zoladex^{®}) und Buserelin (Profact^{®}). Die beschriebenen unerwünschten Arzneimittelnebenwirkungen treten bei einer Anwendung der oben genannten Arzneimittel im Rahmen ihrer üblichen und den Herstellerangaben entsprechenden Verwendung und Dosierung auf.

In einer bevorzugten Ausführungsform umfasst die erfindungsgemäße Zusammensetzung neben mindestens einem Lektin weiterhin mindestens eine Selenverbindung oder/und mindestens ein proteolytisches Enzym sowie gegebenenfalls weitere Hilfs- und Zusatzstoffe.

Jedes mit einer erfindungsgemäßen Zusammensetzung kombinierbares Selen kann verwendet werden. Vorzugsweise ist das Selen ein Natruim-Selenit, ein Kalium-Selenit, ein Magnesium-Selenit, ein Ammonium-Selenit oder/und eine vergleichbare Selenverbindung. Als besonders vorteilhaft hat sich die Verwendung von NatriumSelenit herausgestellt.

Die Kombination eines Lektin's mit einem Selen und einem proteolytischen Enzym hat sich als besonders vorteilhaft erwiesen. Als Enzym kann jedes proteolytische Enzym verwendet werden, das mit den anderen Bestandteilen vereinbar und kombinierbar ist. Besonders vorteilhaft wird ein Papain, ein Bromelain, ein Trypsin oder/und ein Chymotrypsin eingesetzt. Vorteilhafter Weise wird eine Kombination mit zwei proteolytischen Enzymen verwendet.

Diese Ausführungsform der Erfindung hat sich als besonders vorteilhaft erwiesen. Es konnte gezeigt werden, dass bei Verabreichung der Kombination von Lektin, Selen und Enzym der gesamte Komplex der negativen Arzneimittelnebenwirkungen positiv beeinflusst wird. Zum einen zeigte sich bei den Nebenwirkungen betreffend trockene Haut, trockene Augen und Nasenschleimhäute Besserungen für die Betroffenen. Beispielsweise wurde es durch die erfindungsgemäße Verwendung der Kombination für Patientinnen möglich, zumindest für einige Stunden wieder Kontaktlinsen zu tragen. Auch ließ der Hautjuckreiz deutlich nach. Die positive Wirkung der erfindungsgemäßen Behandlung auf alle Aspekte des Nebenwirkungskomplexes führte denn auch zu einer signifikanten Verbesserung der Lebensqualität, da sich eben nicht nur einzelne Symptome verbesserten, sondern die Nebenwirkungssymptomatik in ihrer Gesamtheit. Somit erzielt die Verwendung der erfindungsgemäßen Kombination positive Effekte, die überraschender Weise über die der Einzelkomponenten hinausgehen. Dies führte zu einer spürbaren Verbesserung der gesamten Lebenssituation der Betroffenen mit allen positiven psycho-sozialen Auswirkungen. Insbesondere erhöhte sich die Compliance (Akzeptanz der Antiöstrogenbehandlung) der Patientinnen für die Antiöstrogenbehandlung. Es ist zu betonen, daß selbst eine geringe Verbesserung der Compliance gerade bei einer Behandlung, die sich über viele Jahre erstreckt, nicht unterschätzt werden darf. Eine Veränderung der Compliance wirkt sich gerade bei schwerwiegenden Krankheiten im Negativen wie im Positiven auf den Behandlungserfolg stark aus. Damit erhöht eine erfindungsgemäße Verwendung der Lektinzusammensetzung in ihrer Kombination mit Selen und ein oder mehreren Enzymen mittelbar auch die Krebsbehandlung bzw. die Antiöstrogenbehandlung zur Vermeidung von bzw. zur Reduzierung des Risikos der Rezidivierung und Metastasierung von Krebspatientinnen. Folglich kann die erfindungsgemäße Verwendung einen erheblichen Einfluss auf die Überlebensrate haben, d.h. sie kann dazu beitragen, die Überlebensrate der so behandelten Patientinnen zu erhöhen.

Da auch Frauen in der Peri und Menopause in einen natürlichen Hormonmangel geraten, wurde auch bei diesen Frauen beobachtet, dass sich die Verabreichung einer erfindungsgemäßen Zusammensetzung, insbesondere die Kombination von Lektin, Selen und proteolytischem Enzym positiv auf den hier auftretenden Symptomkomplex auswirkte. Dabei war überraschend, dass es zu einer Besserung nicht nur einzelner Beschwerden kam, sondern dass sich das Gesamtbefinden der Betroffenen spürbar verbesserte. Die erfindungsgemäße Zusammensetzung in ihrer Kombination zeigte also auch hier eine synergistische Wirkung gegenüber den Einzelkomponenten.

Die erfindungsgemäße Zusammensetzung soll in einer Tagesdosis verabreicht werden, deren Anwendung die zu behandelnden unerwünschten Arzneimittelnebenwirkungen wirksam vermindern oder ganz beseitigen kann.

Vorteilhaft in der erfindungsgemäßen Zusammensetzung entspricht die Tagesdosis des Lektin's 2 bis 200 mg, vorzugsweise 5 - 80 mg, besonders bevorzugt 10 - 50 mg und besonders bevorzugt 15 - 30 mg. In einer besonders bevorzugten Ausführungsform beträgt die Tagesdosis 20 mg.

Eine bevorzugte Tagesdosis der Selenverbindung beträgt 30 - 700 mikrogramm, vorzugsweise 50 - 500 mikrogramm oder eine äquimolare Menge einer anderen Selenverbindung. Bevorzugt wird die Selenverbindung in einer Tagesdosis von bis 200 - 400 mikrogramm in der erfindungsgemäßen Zusammensetzung, vorzugsweise von 250 - 350 mikrogramm und in einer weiteren bevorzugten Zusammensetzung von 300 mikrogramm verwendet.

Eine bevorzugte Tagesdosis des proteolytischen Enzym's beträgt 200 - 2000 mg oder entsprechende FIP-Einheiten, beispielsweise 1000 - 10000 FIP-Einheiten. Bevorzugte weitere Bereiche sind 300 - 1500 mg, vorzugsweise 500 - 1200 mg und weiterhin bevorzugt 800 mg bzw. die entsprechenden FIP-Einheiten.

Es liegt im Verständnis des Fachmann's die genannten bevorzugten Bereiche der Einzelbestandteile in einer erfindungsgemäßen Kombination in jeder geeigneten und vorteilhaften Weise miteinander zu kombinieren.

Die erfindungsgemäße Zusammensetzung kann in jeder geeigneten Darreichungsform zubereitet werden. Vorzugsweise ist die Zusammensetzung zubereitet als eine Tablette, eine Retardtablette, eine Kapsel, ein Dragee, Pulver, Granulat oder eine Suspension.

Eine bevorzugte Zusammensetzung umfaßt 5 mg Lektin, vorzugsweise Lens culinaris, 100 mg Papain, 100 mg Bromelain, 50 oder 75 mikrogramm Natriumselenit sowie als Zusatz- und Hilfsmittel Reisstärke, Magnesiumstearat und Schellack.

In einer weiteren bevorzugten Ausführungsform umfasst die Zusammensetzung 5 mg Lektin, vorzugsweise Lens culinaris, 140 mg Papain, 60 mg Bromelain, 50 microgramm Natriumselenit sowie als Zusatz und Hilfsmittel Reisstärke und Magnesiumstearat.

Als Hilfs- und Trägerstoffe kommen alle dem Fachmann und mit der speziellen Kombination der Einzelbestandteile vereinbaren bekannten Stoffe in Frage.

In einer weiteren bevorzugten Ausführungsform wird die erfindungsgemäße Zusammensetzung in einer Tagesdosis eingenommen, die 20 mg Lektin, vorzugsweise Lens culinaris, 400 mg Papain, 400 mg Bromelain, 300 microgramm Natriumselenit umfaßt.

### Beispiele

Die folgenden Beispiele stellen bevorzugte Ausgestaltungen der Erfindung dar. Sie sollen die Erfindung illustrieren ohne beschränkend verstanden zu werden.

| **1. Lektinzusammensetzung Tablette:** | | **A.** | **B.** |
|---|---|---|---|
| Lektin: | Lens culinaris | 5.0 mg | 5.0 mg |
| Selenverbindung: | Natriumselenit | 0.075 mg | 0.050 mg |
| Enzym: | Papain | 100 mg | 140 mg |
| | Bromelain | 100 mg | 60 mg |
| | | | |
| Hilfs- und Zusatzstoffe: | Reisstärke | ja | ja |
| | Magnesiumstearat | ja | ja |
| | Schellack | ja | nein |

### 2. Behandlung von durch Zoladexbehandlung induzierter Arzneimittelnebenwirkung mittels einer erfindungsgemäßen ergänzend bilanzierten Diät (ebD)

Eine Brustkrebspatientin wurde nach Operation und Chemotherapie einer Antihormontherapie (Tamoxifen+Gonadotropin Releasing Hormon = GnRh-Analoga Zoladex) unterzogen. Dabei wurden die vom Hersteller vorgegebenen Behandlungsdosen verwendet. Im Verlaufe dieser mehrjährigen Behandlung kam es zu erheblichen Nebenwirkungen: Gelenkschmerzen in Knie, Sprunggelenk, Schultergelenk und den Fingern. Einfache Bewegungen wie Treppensteigen oder das Halten von Messer und Gabel waren stark eingeschränkt und mit erheblichen Schmerzen verbunden. Das Tragen von Kontaktlinsen wurde aufgrund fehlender Augenflüssigkeit unmöglich. Nasen-, Mund- und Scheidenschleimhaut entzündeten sich immer wieder. Der Komplex der arzneimittelbedingten Nebenwirkungen führte zu einem erheblichen Verlust der Lebensqualität für die Patientin verbunden mit psychischen Begleiterscheinungen.

Die Patientin erhielt täglich zwei mal zwei Tabletten vom erfindungsgemäßen Typ A (siehe oben unter 1). Bereits nach drei Tagen war eine deutliche und spürbare Milderung der arzneimittelbedingten Nebenwirkungen von der Patientin wahrnehmbar. Die Patientin konnte zumindest für Stunden wieder Kontaktlinsen tragen, da nach der erfindungsgemäßen Behandlung ausreichend Tränenflüssigkeit in den Augen vorhanden war. Die Schleimhautentzündungen gingen deutlich zurück. Die Finger sowie die anderen Gelenke konnten wieder schmerzfrei bewegt werden. Insgesamt war eine wesentliche Steigerung der Lebensqualität für die Patientin feststellbar, verbunden mit psychischer Stabilisierung.

Beim Absetzen der erfindungsgemäßen ergänzend bilanzierten Diät nach einem Jahr stellten sich die oben genannten arzneimittelbedingten Nebenwirkungen wieder ein. Nach erneuter erfindungsgemäßer Behandlung konnten die genanten Nebenwirkungen wieder vermindert oder beseitigt werden.

Die Behandlung mit der erfindungsgemäßen ergänzend bilanzierten Diät wurde daraufhin während der gesamten Antihormontherapie fortgesetzt.

### 3. Behandlung von durch Tamoxifenbehandlung induzierter Arzneimittelnebenwirkung mittels einer erfindungsgemäßen ergänzend bilanzierten Diät (ebD)

Die Antiöstrogen-Behandlung (Tamoxifen) einer Krebspatientin mit den vom Hersteller angegebenen Dosen führte zu erheblichen Arzneimittelbedingten Nebenwirkungen. Diese waren vor allem gekennzeichnet durch Gelenkschmerzen. Diese Nebenwirkungen waren derart schwerwiegend, dass die Patientin trotz der möglichen negativen Implikationen erwägte, die Antiöstrogen-Behandlung abzubrechen. Die Patientin erhielt daraufhin zweimal täglich zwei Tabletten vom erfindungsgemäßen Typ A (siehe oben unter 1) parallel zu der Tamoxifenbehandlung. Nach einer Woche der erfindungsgemäßen Behandlung stellte sich eine deutliche Milderung der arzneimittelbedingten Nebenwirkungen ein und die Antiöstrogenbehandlung konnte erfolgreich fortgesetzt werden.

### 4. Behandlung von durch Letrozol induzierter Arzneimittelnebenwirkung mittels einer erfindungsgemäßen ergänzend bilanzierten Diät (ebD)

Eine Patientin wurde einer Letrozolbehandlung mit den vom Hersteller angegebenen Dosen unterzogen. Dabei kam es zu erheblichen arzneimittelbedingten Nebenwirkungen wie Gelenkschmerzen, Trockenheit der Schleimhäute. Die Patientin erhielt daraufhin zweimal täglich eine Tablette vom erfindungsgemäßen Typ A (siehe oben unter 1) parallel zu der Letrozolbehandlung. Nach einer Woche der erfindungsgemäßen Behandlung stellte sich eine deutliche Milderung der arzneimittelbedingten Nebenwirkungen ein, die die Lebensqualität (quality of life, QoL) der Patientin nachhaltig verbesserte.

Es kann davon ausgegangen werden, dass die Verwendung einer Tablette vom Typ B (siehe oben unter 1.) mit der entsprechenden Dosierung vergleichbare positive Ergebnisse ergibt.

### 5. Behandlung von unerwünschten Arzneimittelnebenwirkungen mit Einzelsubstanzen und Substanzkombinationen gemäß der Erfindung

Mit Antihormontherapie Tamoxifen (n=34), Femara (n=21), Arimidex (n=16), Aromasin (N=8) oder Zoladex (N=5) behandelte Patientinnen wurden nach dem Zufallsprinzip in verschiedene Behandlungsgruppen eingeteilt. Die Antihormonbehandlung beginnt im Allgemeinen mit dem Hormonblocker Tamoxifen und wird nach 2-3 Jahren weitergeführt mit Tamoxifen oder ersetzt durch die Aromatasehemmertherapie (Femara, Arimidex, Aromasin). Junge Frauen mit Krebs erhalten häufig das GnRH Analogon Zoladex. Die in die Studie aufgenommenen Patientinnen waren bereits zwischen 1,5 und 7,2 Jahren unter Antihormontherapie (im Mittel 3,7 Jahre). Diese Patientinnen litten an einer Anzahl von unerwünschten arzneimittelbedingten Nebenwirkungen in unterschiedlicher Schwere.

Jede Gruppe erhielt parallel zu der Antihormontherapie täglich eine Behandlung mit den einzelnen erfindungsgemäßen Bestandteilen wie Lektinen, Enzymen, Na-Selenit oder eine Kombination dieser Bestandteile wie folgt:
**Gruppe I:** Antihormontherapie ohne parallele Behandlung (Kontrollgruppe I),
**Gruppe II:** 4 x täglich nur 5 mg Lektin (Lens culinaris) (Tagesdosis: 20 mg Lektin) parallel zur Antihormontherapie.
**Gruppe III:** 4 x täglich nur Enzyme: jeweils 100 mg Bromelain + 100 mg Papain (Tagesdosis: 400 mg Bromelain, 400 mg Papain) parallel zur Antihormontherapie,
**Gruppe IV:** 4 x täglich nur Na-Selenit: jeweils 75 microgram Na-Selenit (Tagesdosis: 300 mg) parallel zur Antihormontherapie,
**Gruppe V:** 4 x täglich 1 Tablette vom Typ A (siehe unter Punkt 1.) (Tagesdosis: Lektin 20 mg Lens culinaris, Enzyme 400 mg Papain und 400 mg Bromelain, Na-Selenit 300 µg) parallel zur Antihormontherapie,
**Gruppe VI:** 2 x täglich 1 Tablette vom Typ A (siehe unter Punkt 1.) (Tagesdosis: Lektin 10 mg Lens culinaris, Enzyme 200 mg Papain und 200 mg Bromelain, Na-Selenit 150 µg) parallel zur Antihormontherapie.

Vor der parallelen Zusatzbehandlung mittels der ergänzend bilanzierten Diät (ebD) wurden die verschiedenen unerwünschten arzneimittelbedingten Nebenwirkungen bei jeder Patientin mittels einer Skala von 1 bis 6 ermittelt (1: praktisch keine Nebenwirkung, 2: merkliche Nebenwirkung, 3: Nebenwirkung sehr unangenehm, 4: sehr starke Nebenwirkung, 5: Nebenwirkung sehr stark bis unerträglich stark, 6: Nebenwirkung unerträglich stark). Hieraus wurde der Gruppenmittelwert für jede der beurteilten Nebenwirkungen gebildet.

Zu Behandlungsbeginn und nach einer Behandlungsdauer von 30 Tagen wurden erneut bei jeder Patientin die unerwünschten arzneimittelbedingten Nebenwirkungen mittels der oben beschriebenen Skala von 1 bis 6 bewertet und wie beschrieben in jeder Gruppe für jede der beurteilten Nebenwirkung der Mittelwert errechnet.

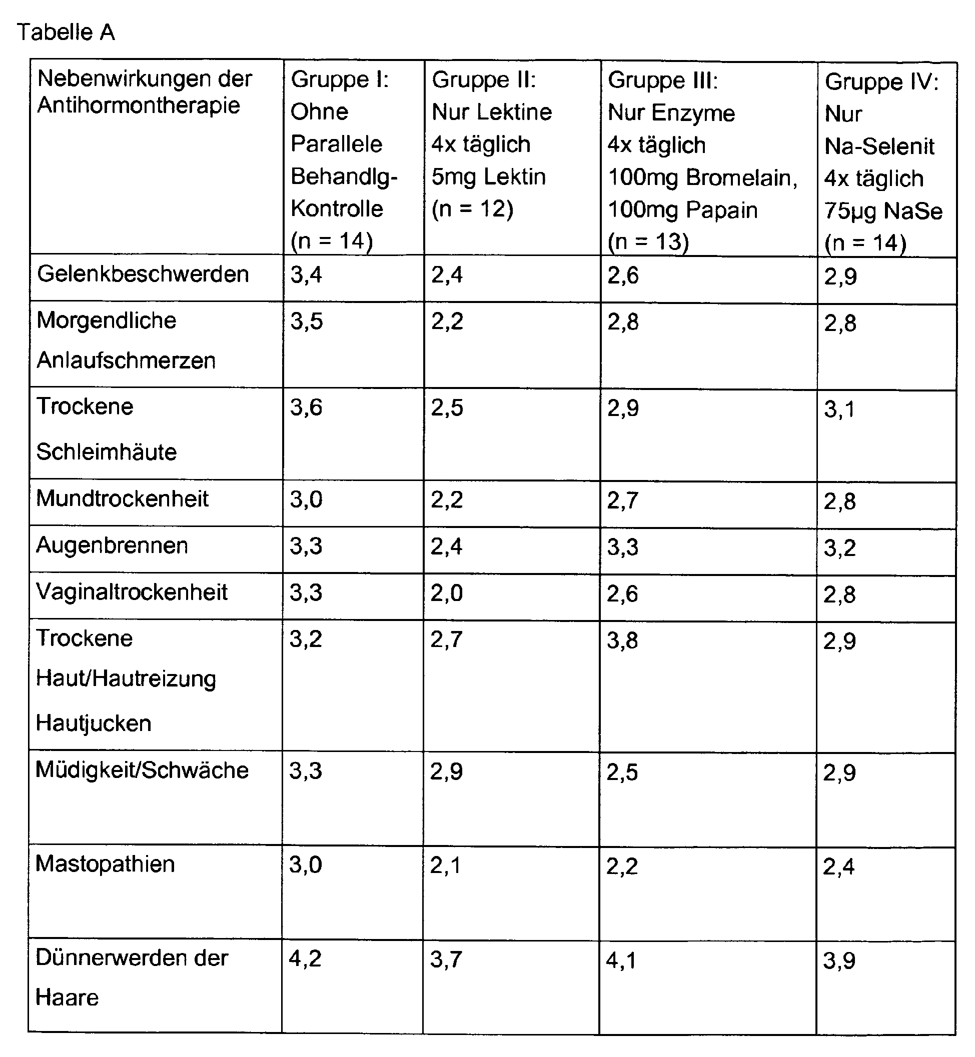

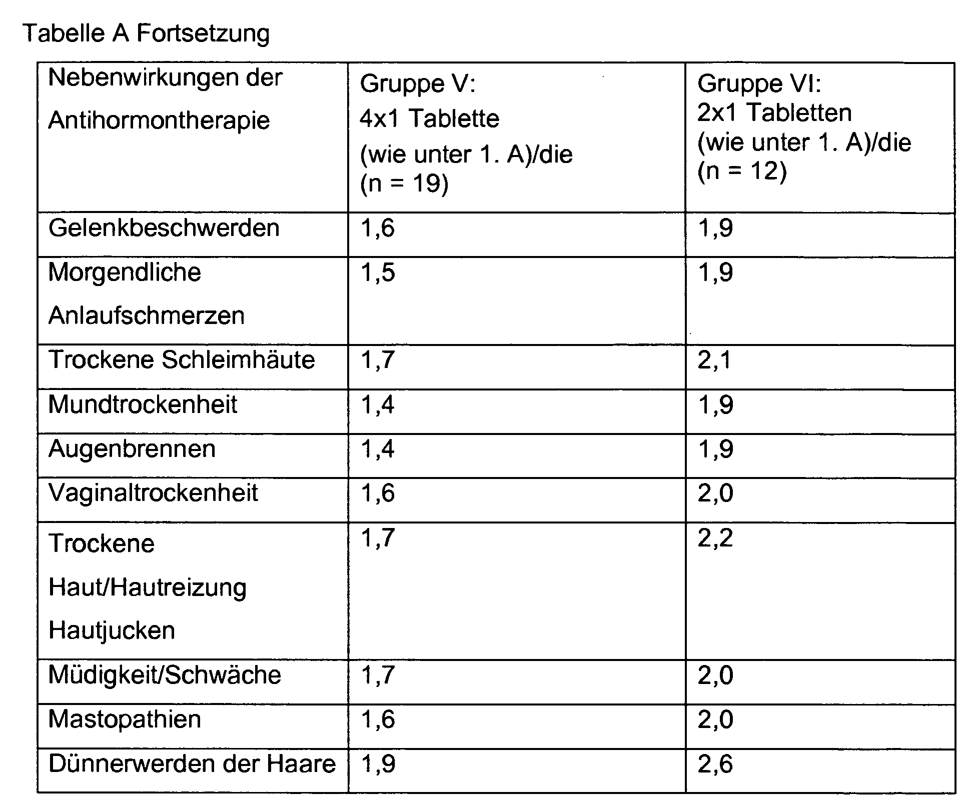

Vor der erfindungsgemäßen Behandlung klagten die Patientinnen über sehr unangenehme bis sehr starke (Bewertungsskala 3 bzw. 4) durch die Antihormontherapie verursachte Nebenwirkungen.

Die gemessenen Nebenwirkungen betrafen dabei ästhetische Aspekte wie das Dünnerwerden der Haare, Nebenwirkungen, die das allgemeine Wohlbefinden betreffen wie Mundtrockenheit bis hin zu Nebenwirkungen, die das tägliche Leben nachhaltig negativ beinträchtigen wie morgendliche Anlaufschmerzen.

Es konnte gezeigt werden, dass bei einer Antihormontherapie eine parallele erfindungsgemäße Behandlung mit Lektinen (Gruppe II) eine deutliche Verringerung der unerwünschten Arzneimittelnebenwirkungen erzielen konnte. Es konnte eine Verbesserung der Nebenwirkungen um durchschnittlich einen Bewertungspunkt der Bewertungsskala erzielt werden und so die Nebenwirkungssituation für die Patientinnen signifikant verbessert werden.

Eine Verringerung der unerwünschten Arzneimittelnebenwirkungen konnte auch für eine Anzahl der unerwünschten Arzneimittelnebenwirkungen durch Einzelgabe von Enzymen (Gruppe III) sowie durch Einzelgabe von Na-Selenit (Gruppe IV) im Vergleich mit der Kontrollgruppe erreicht werden.

Somit ist es möglich, mittels einer erfindungsgemäßen Behandlung bestehend aus Einzelgaben von Lektinen, vorzugsweise Enzymen und Na-Selenit unerwünschte Arzneimittelnebenwirkungen für die Patientinnen deutlich zu verbessern und dadurch die Lebensqualität (quality of life, QoL) signifikant zu erhöhen.

Weiterhin zeigen die von den Erfindern durchgeführten Untersuchungen, dass überraschender Weise die erfindungsgemäße Verwendung einer Kombination von Lektinen, Enzymen und Na-Selenit (Gruppe V und Gruppe VI) im Vergleich zu den Einzelbestandteilen eine über die Einzelbestandteile hinausgehende positive synergistische Wirkung für die QoL der getesteten Patientinnen erreichen konnte.

In Gruppe V (Tagesdosis: Lektin 20 mg Lens culinaris, Enzyme 400 mg Papain und 400 mg Bromelain, Na-Selenit 300 µg) konnten mittels der erfindungsgemäßen Behandlung die unerwünschten arzneimittelbedingten Nebenwirkungen von 3 bis 4 auf der Bewertungsskala (siehe Kontrollgruppe I) auf Werte von 1,4 bis 1,9 verringert werden. Die Patientinnen litten also vor der erfindungsgemäßen Behandlung an sehr unangenehmen (Bewertungsskala ca. 3) bis sehr starken (Bewertungsskala ca. 4) Nebenwirkungen und zeigten nach der erfindungsgemäßen Behandlung wenn überhaupt nur noch leichte Nebenwirkungen (Bewertungsskala ca. 1). Sie waren somit praktisch nebenwirkungsfrei.

Ein derart positiver Behandlungserfolg mittels der erfindungsgemäßen Behandlung in Einzelgabe von Lektinen und besonders der erfindungsgemäßen Kombination von Lektinen, Enzymen und Na-Selenit war überraschend und nicht vorhersehbar. Als besonders vorteilhaft hat sich eine erfindungsgemäße Behandlung in den beschriebenen Tagesdosen in den durchgeführten und beschriebenen Versuchen gezeigt.

Auch eine Reduzierung der Tagesdosis der Substanzkombination um die Hälfte (Gruppe VI, Tagesdosis: Lektin 10 mg Lens culinaris, Enzyme 200 mg Papain und 200 mg Bromelain, Na-Selenit 150 µg) zeichnete sich weitgehend durch eine synergistische Wirkung gegenüber den Einzelbestandteilen aus. Auch in dieser Gruppe konnten die unerwünschten arzneimittelbedingten Nebenwirkungen deutlich reduziert werden. Hier zeigte sich eine Dosisabhängigkeit der Tagesdosis der erfindungsgemäßen Behandlung. Die Werte der Nebenwirkungen gemäß Bewertungsskala fielen bei dieser Dosis gleichermaßen deutlich geringer aus im Vergleich zu beispielsweise der Einzelbehandlung mit Lektinen. Dies ist ein weiterer Beleg für die positive und synergistische Wirkung der erfindungsgemäßen Kombination der Einzelbestandteile. Weiterhin machen die positiven Ergebnisse mittels verschiedener Tagesdosisbereiche deutlich, dass die erfindungsgemäße Wirkstoffkombination über einen großen Dosisbereich und in verschiedenen Tagesdosen wirksam ist. Die bevorzugten Bereiche haben sich dabei als besonders vorteilhaft erwiesen.

## Patentansprüche

1. Lektinzusammensetzung zur Anwendung zur Prophylaxe oder/und Behandlung von Aromatase-Hemmer -, Antiöstrogen - oder/und GnRH-Analoga - bedingten unerwünschten Arzneimittelnebenwirkungen oder von durch einen endogenen Östrogendefizit in der Menopause bei Frauen - bedingten Beschwerdekomplex in einem Individuum.

2. Zusammensetzung nach Anspruch 1, **dadurch gekennzeichnet, dass** die Lektinzusammensetzung eine pharmazeutische Zusammensetzung, ein diätetisches Lebensmittel, ein Lebensmittel für die bilanzierte Diät, eine Nahrungsergänzungszusammensetzung oder/und Medizinprodukt ist.

3. Zusammensetzung nach einem der vorhergehenden Ansprüche, **dadurch gekennzeichnet, dass** das Lektin ein pflanzliches oder tierisches Lektin ist.

4. Zusammensetzung nach Anspruch 3, **dadurch gekennzeichnet, dass** das Lektin ein Lens culinaris oder ein Pisum sativum ist.

5. Zusammensetzung nach einem der vorhergehenden Ansprüche, **dadurch gekennzeichnet, dass** der Aromatase-Hemmer ausgewählt ist aus der Gruppe bestehend aus Aminoglutethimid, Anastrozol (Arimidex), Letrozol (Femara), Vorozol (Rivizor), Exemestan (Aromasin), 1,4,6-androstatrien-3,17-dione ATD), Formestan (Lentaron), Testolactone (Teslac) und Fadrozol (Afeman).

6. Zusammensetzung nach einem der vorhergehenden Ansprüche, **dadurch gekennzeichnet, dass** das Antiöstrogen oder der Östrogenrezeptormodulator (SERM) ausgewählt ist aus der Gruppe bestehend aus Clomifen, Raloxifen, Tamoxifen, Toremifen, Bazedoxifen, Lasofoxifen und Ormeloxifen.

7. Zusammensetzung nach einem der vorhergehenden Ansprüche, **dadurch gekennzeichnet, dass** das GnRH-Analoga ausgewählt ist aus der Gruppe bestehend aus Leuprorelin (Eligard^{®}, Enantone^{®}), Goserelin (Zoladex^{®}) und Buserelin (Profact^{®}).

8. Zusammensetzung nach einem der vorhergehenden Ansprüche, **dadurch gekennzeichnet, dass** die Zusammensetzung weiterhin umfasst mindestens eine Selenverbindung oder/und mindestens ein proteolytisches Enzym sowie weitere Hilfs- und Zusatzstoffe.

9. Zusammensetzung nach Anspruch 6, **dadurch gekennzeichnet, dass** das Selen ein Natruim-Selenit, ein Kalium-Selenit, ein Magnesium-Selenit, ein Ammonium-Selenit oder/und eine vergleichbare Selenverbindung ist.

10. Zusammensetzung nach Anspruch 6, **dadurch gekennzeichnet, dass** das proteolytische Enzym ein Papain, ein Bromelain, ein Trypsin oder/und ein Chymotrypsin ist.

11. Zusammensetzung nach einem der vorhergehenden Ansprüche, **dadurch gekennzeichnet, dass** die Tagesdosis des Lektin's mindestens 2 - 100, vorzugsweise 10 - 50 mg entspricht.

12. Zusammensetzung nach einem der vorhergehenden Ansprüche, **dadurch gekennzeichnet, dass** die Tagesdosis der Selenverbindung 30 - 700, vorzugsweise 50 - 500 mikrogramm oder eine äquimolaren Menge einer anderen Selenverbindung entspricht.

13. Zusammensetzung nach einem der vorhergehenden Ansprüche, **dadurch gekennzeichnet, dass** die Tagesdosis des proteolytischen Enzym's 200 - 2000 mg oder 1000 - 10000 FIP-Einheiten, vorzugsweise 600 - 1200 mg, entspricht.

14. Zusammensetzung nach einem der vorhergehenden Ansprüche, **dadurch gekennzeichnet, dass** die Zusammensetzung zubereitet ist als eine Tablette, eine Retardtablette, eine Kapsel, ein Dragee, Pulver, Granulat oder eine Suspension.

15. Zusammensetzung nach einem der vorhergehenden Ansprüche, **dadurch gekennzeichnet, dass** die Zusammensetzung umfasst 5 mg Lektin, vorzugsweise Lens culinaris, 100 oder 140 mg Papain, 100 oder 60 mg Bromelain, 50 oder 75 microgramm Natriumselenit sowie als Zusatz und Hilfsmittel Reisstärke, Magnesiumstearat und Schellack.

## Claims

1. Lectin composition for use in the prophylaxis or/and treatment of undesired drug side effects caused by aromatase inhibitors, anti-estrogens or/and GnRH analoga, or of a discomfort complex caused by an endogenous estrogen deficit during the menopause in women in a subject.

2. Composition according to claim 1, **characterized in that** the lectin composition is a pharmaceutical composition, dietetic food, foodstuffs for the balanced diet, a dietary supplement composition or/and a medicinal product.

3. Composition according to any one of the preceding claims, **characterized in that** the lectin is a plant or animal lectin.

4. Composition according to claim 3, **characterized in that** the lectin is from lens culinaris or from a pisum sativum.

5. Composition according to any one of the preceding claims, **characterized in that** the aromatase inhibitor is selected from the group consisting of aminoglutethimide, Anastrozole (Arimidex), Letrozole (Femara), Vorozole (Rivizor), Exemestane (Aromasin), 1,4,6-androstatrien-3,17-dione (ATD), Formestane (Lentaron), Testolactone (Teslac) und Fadrozole (Afeman).

6. Composition according to any one of the preceding claims, **characterized in that** the anti-estrogen or the estrogen receptor modulator (SERM) is selected from the group consisting of Clomifene, Raloxifene, Tamoxifene, Toremifene, Bazedoxifene, Lasofoxifene and Ormeloxifene.

7. Composition according to any one of the preceding claims, **characterized in that** the GnRH analoga is selected from the group consisting of Leuproreline (Eligard^{®}, Enantone^{®}), Gosereline (Zoladex^{®}) and Busereline (Profact^{®}).

8. Composition according to any one of the preceding claims, **characterized in that** the composition further comprises at least one selenium compound or/and at least one proteolytic enzyme as well as further supplementary and additive compounds.

9. Composition according to claim 6, **characterized in that** the selenium is a sodium selenite, a potassium selenite, a magnesium selenite, an ammonium selenite or/and a comparable selenium compound.

10. Composition according to claim 6, **characterized in that** the proteolytic enzyme is a papain, a bromelin, a trypsin or/and a chymotrypsin.

11. Composition according to any one of the preceding claims, **characterized in that** the daily dosis of the lectin equals at least 2 - 100, preferably 10 - 50 mg.

12. Composition according to any one of the preceding claims, **characterized in that** the daily dosis of the selenium compound equals 30 - 700, preferably 50 - 500 microgram or it equals an equimolar amount of another selenium compound.

13. Composition according to any one of the preceding claims, **characterized in that** the daily dosis of the daily dosis of the proteolytic enzyme equals 200 - 2000 mg or 1000 - 10000 FIP-units, preferably 600 - 1200 mg.

14. Composition according to any one of the preceding claims, **characterized in that** the composition is readily prepared as a tablet, a retard tablet, a capsule, a coated tablet, a powder, a granulate or a suspension.

15. Composition according to any one of the preceding claims, **characterized in that** the composition comprises 5 mg Lectin, preferably lens culinaris, 100 or 140 mg papain, 100 or 60 mg bromelin, 50 or 75 microgram sodium selenite as well as as supplementary and additive compounds rice starch, magnesium stearate and schellack.

## Revendications

1. Composition de lectine destinée à être utilisée pour la prophylaxie ou/et le traitement, chez l'individu, ou bien d'effets secondaires indésirables d'un médicament produits par les inhibiteurs d'aromatase, les anti-oestrogènes ou/et les analogues du GnRH ou bien d'un ensemble de maladies résultant d'un déficit d'oestrogène endogène chez les femmes ménopausées.

2. Composition selon la revendication 1, **caractérisée en ce que** ladite composition de lectine est une composition pharmaceutique, un aliment diététique, un aliment pour les régimes à apport énergétique contrôlé, un complément alimentaire ou/et un produit médical.

3. Composition selon l'une quelconque des revendications précédentes, **caractérisée en ce que** ladite lectine est d'origine végétale ou animale.

4. Composition selon la revendication 3, **caractérisée en ce que** ladite lectine est du type Lens culinaris ou Pisum sativum.

5. Composition selon l'une quelconque des revendications précédentes, **caractérisée en ce que** ledit inhibiteur d'aromatase est choisi dans le groupe constitué par l'aminoglutéthimide, l'anastrozole (Arimidex), le létrozole (Femara), le vorozole (Rivizor), l'exemestane (Aromasin), le 1,4,6-androstatriène-3,17-dione (ATD), le formestane (Lentaron), le testolactone (Teslac) et le fadrozole (Afeman).

6. Composition selon l'une quelconque des revendications précédentes, **caractérisée en ce que** ledit anti-oestrogène ou le modulateur sélectif des récepteurs aux oestrogènes (SERM) est choisi dans le groupe constitué par le clomifène, le raloxifène, le tamoxifène, le torémifène, le bazedoxifène, le lasofoxifène et l'ormeloxifène.

7. Composition selon l'une quelconque des revendications précédentes, **caractérisée en ce que** ledit analogue du GnRH est choisi dans le groupe constitué par la leuproréline (Eligard^{®}, Enantone^{®}), la goséréline (Zoladex^{®}) et la buséréline (Profact^{®}).

8. Composition selon l'une quelconque des revendications précédentes, **caractérisée en ce que** ladite composition comprend en outre au moins un composé de sélénium ou/et au moins une enzyme protéolytique ainsi que d'autres agents auxiliaires et additifs.

9. Composition selon la revendication 6, **caractérisée en ce que** ledit composé de sélénium est un sélénite de sodium, un sélénite de potassium, un sélénite de magnésium, un sélénite d'ammonium ou/et un composé de sélénium similaire.

10. Composition selon la revendication 6, **caractérisée en ce que** ladite enzyme protéolytique est une papaïne, une bromélaïne, une trypsine ou/et une chymotrypsine.

11. Composition selon l'une quelconque des revendications précédentes, **caractérisée en ce que** la dose journalière de ladite lectine correspond à au moins 2-100 et de préférence à 10-50 mg.

12. Composition selon l'une quelconque des revendications précédentes, **caractérisée en ce que** la dose journalière dudit composé de sélénium correspond à 30-700, de préférence à 50-500 microgrammes, ou à une quantité équimolaire d'un autre composé de sélénium.

13. Composition selon l'une quelconque des revendications précédentes, **caractérisée en ce que** la dose journalière de ladite enzyme protéolytique correspond à 200-2000 mg ou 1000-10.000 unités FIP, de préférence à 600-1200 mg.

14. Composition selon l'une quelconque des revendications précédentes, **caractérisée en ce que** ladite composition est préparée sous la forme d'un comprimé, d'un comprimé à libération prolongée, d'une gélule, d'une dragée, d'une poudre, d'un granulé ou d'une suspension.

15. Composition selon l'une quelconque des revendications précédentes, **caractérisée en ce que** ladite composition comprend 5 mg de lectine, de préférence Lens culinaris, 100 ou 140 mg de papaïne, 100 ou 60 mg de bromélaïne, 50 ou 75 microgrammes de sélénite de sodium ainsi que des additifs et agents auxiliaires sous la forme de l'amidon de riz, du stéarate de magnésium et de la gomme-lacque.
